# EUROPÄISCHE PATENTSCHRIFT

(11) **EP 1 518 475 B1**
(45) Veröffentlichungstag und Bekanntmachung des Hinweises auf die Patenterteilung: **23.01.2008**
(21) Anmeldenummer: 04104760.6
(22) Anmeldetag: 29.09.2004
(51) Int. Cl.: A45D 20/12, A61L 9/03

(54) **Haarform- oder Haarpflegegerät**
Hair styling or hair care device
Appareil de mise en forme ou de soins des cheveux

(30) Priorität: 29.09.2003 DE 20315128 U
(43) Veröffentlichungstag der Anmeldung: 30.03.2005
(73) Patentinhaber: WIK Far East Ltd., North Point, Hong Kong (CN)
(72) Erfinder: Hafemann, Klaus, 45359 Essen (DE)
(74) Vertreter: Haverkamp, Jens

(56) Entgegenhaltungen:
- US-A- 5 572 800
- US-A- 5 987 771
- US-A1- 2003 159 306

## Beschreibung

Die Erfindung betrifft ein elektrisches Haarform- oder Haarpflegegerät mit einem Warmluftgebläse zum Fördern eines für eine Haarbehandlung - Trocknen und/oder Formen - notwendigen Warmluftstroms, wobei das Gerät ein oder mehrere, von dem geförderten Luftstrom angeströmte, Duft abgebende Elemente aufweist und die von einem solchen Element bei einem Betrieb des Gerätes abgegebenen, sensorisch aktiven Moleküle von dem geförderten Luftstrom aufgenommen und zum Haar transportiert werden

Bei derartigen Haarform- oder Haarpflegegeräten handelt es sich um Haartrockner oder um Haarformgeräte beispielsweise Frisierstäbe, Lockenwickler, ausgerüstet jeweils mit einem Warmluftgebläse. Diesen Geräten ist gemein, dass für die gewünschte Pflege der Haare, etwa zum Trocknen oder zum Formen ein Warmluftstrom eingesetzt wird. Dieser ist bei einem Haartrockner unmittelbar auf das zu trocknende Haar oder bei einem Haarformer unter Zwischenschaltung unterschiedlicher Haarformelemente, die beispielsweise als Aufsatz ausgebildet sein können, mittelbar auf das Haar gerichtet. In jedem Fall erreicht ein von dem Warmluftgebläse geförderter Warmluftstrom das Haar. In Abhängigkeit von dem jeweiligen Pflege- oder Formprozess kann es vorkommen, dass die Haare einen charakteristischen, diesen Geräten eigenen Geruch annehmen, der sich beispielsweise dann einstellt, wenn Staubteilchen oder Haare an der dem Warmluftgebläse zugeordneten Heizeinrichtung verbrannt werden. Dieser Geruch kann von den Haaren angenommen werden. Für den Fall, dass man nicht abwarten möchte, bis dieser Geruch verflogen ist, ist eine Nachbehandlung der Haare im Wege einer Parfümierung notwendig. Das Parfum wird als Haarspray oder mittels eines Parfümzerstäubers appliziert, was jedoch zum Nachteil hat, dass eine solche Nachbehandlung feuchtigkeitsbasiert ist und daher die zuvor unter Umständen mit Aufwand erstellte Frisur beschädigen kann.

Darüber hinaus ist aus US 4 615 347 A ein Haartrockner bekannt geworden, auf deren Luftaustrittsöffnung zum Auslassen des geförderten Warmluftstroms ein Aufsatz mit einem sich über die Querschnittsfläche des austretenden Warmluftstroms erstreckenden Gitter aufsteckbar ist. An den Knotenpunkten des Gitteraufsatzes sind Duft abgebende Kügelchen angeordnet. Somit verfügt dieser Aufsatz über eine der Anzahl der Gitterknotenpunkte entsprechende Anzahl Duft abgebender Elemente. Der an diesen Duft abgebenden Elementen vorbeiströmende Warmluftstrom nimmt von diesen abgegebene sensorisch aktive Moleküle auf und transportiert diese an das zu pflegende oder zu formende Haar, so dass eine auf Feuchtigkeit basierte Nachbehandlung des Haars grundsätzlich nicht notwendig ist.

Nachteilig ist bei diesem Stand der Technik jedoch, dass die Duftabgabemenge der Duft abgebenden Elemente nicht eingerichtet oder eingestellt werden kann. Typischerweise ist die Duftabgabemenge bei einem Einsatz eines neuen Aufsatzes größer und nimmt über die Benutzungsdauer ab. Des Weiteren kann es gewünscht sein, die Haare unterschiedlicher Personen mit unterschiedlicher Duftintensität zu parfümieren.

Ein weiterer Haartrockner mit einem Duft abgebenden Element ist aus der US 5 987 771 A bekannt.

Vor diesem aufgezeigten Stand der Technik liegt der Erfindung die Aufgabe zugrunde, ein eingangs genanntes, gattungsgemäßes Haarformoder -pflegegerät dergestalt weiterzubilden, dass die Duftabgabemenge seines Duft abgebenden Elements grundsätzlich eingestellt werden kann.

Diese Aufgabe wird erfindungsgemäß durch ein eingangs genanntes, gattungsgemäßes Haarform- oder -pflegegerät gelöst, bei dem das Temperaturprofil innerhalb des geförderten Warmluftstroms über sein Querschnittsprofil hinweg im Bereich des Duft abgebenden Elements ein oder mehrere Zonen tieferer Temperaturen und zumindest eine Zone höherer Temperatur aufweist, die Duftabgabemenge des Duft abgebenden Elements bei steigenden Temperaturen zunimmt und das Duft abgebende Element ausgebildet ist, damit in Abhängigkeit von der Anordnung des Duft abgebenden Elements innerhalb des geförderten Warmluftstroms unter Ausnutzung der unterschiedlichen Temperaturzonen des Warmluftstroms die Duftabgabemenge unterschiedlich ist.

Dieses Haarform- oder Haarpflegegerät verfügt über ein einen Duftstoff oder ein Duftstoffgemisch abgebendes Element, das innerhalb des von dem Gebläse geförderten Luftstroms angeordnet ist oder an dem der von dem Warmluftgebläse geförderte Luftstrom entlangstreicht. Mithin ist das Duft abgebende Element von dem Warmluftstrom angeströmt. Dieses Duft abgebende Element nimmt bei einem Betrieb des Haarform- oder Pflegegerätes sensorisch aktive Moleküle - Duftmoleküle - auf und transportiert diese in das Haar, auf das der Luftstrom gerichtet ist. Die Duftmoleküle, die von einem solchen Duft abgebenden Element abgegeben - unter Temperatureinfluss ausgeschwitzt - werden, sind relativ klein und lassen sich daher besonders gut mit dem geförderten Luftstrom transportieren, insbesondere auch durch ein Haarformgerät, beispielsweise eine Bürste hindurch bis an das zu behandelnde Haar. An diesem lagern sich die sensorisch aktiven Moleküle an, so dass mit der abgeschlossenen Haarpflege oder Haarformbehandlung die Haare angenehm duften. Ein solches Duft abgebendes Element kann mit Duftstoffen unterschiedlichster Geruchsrichtung dotiert sein.

Bei einem solchen Duft abgebenden Element handelt es sich zweckmäßigerweise um ein Kunststoffformteil als Träger für den Duftstoff, wobei unterschiedlichste Duftstoffe oder auch Duftstoffgemische in einem solchen Kunststoffformteil bevorratet werden können. Das Kunststoffformteil übernimmt in diesem Fall die Funktion eines Schwammes, von dem nach und nach Duftmoleküle abgegeben werden, wobei in einem bevorzugten Ausführungsbeispiel vorgesehen ist, dass eine Duftmolekülabgabe im nennenswerten Umfange nur unter Temperatureinfluss und somit bei einem Betrieb des Gerätes zum Fördern eines Warmluftstromes erfolgt. Die Duftabgabemenge eines solchen Duft abgebenden Kunststoffs ist grundsätzlich abhängig von der Temperatur des anströmenden Luftstroms, wobei mit steigender Temperatur auch die abgegebene Duftmenge und somit die Duftmolekülkonzentration steigt. Ein solches Duft abgebendes Element, insbesondere als Kunststoffformteil ausgebildet, muss nicht notwendigerweise ein zusätzliches Element sein; vielmehr kann einem dem Haarform- oder Haarpflegegerät ohnehin zugehöriges Element aus einem solchen Formteil bestehen oder das Duft abgebende Element ist Teil eines solchen Formteils. Das im Bereich des Luftaustritts angeordnete Formteil kann neben seiner Duft abgebenden Funktion auch andere funktionelle Eigenschaften übernehmen, beispielsweise wenn dieses als Aufsatz, etwa als Ondulierdüse oder dergleichen ausgebildet ist.

Aus dem Vorbeschriebenen wird deutlich, dass das Duft abgebende Element Teil eines Haartrockners oder auch eines Harrformgerätes sein kann und dass dieses insbesondere austauschbar konzipiert sein kann. Im Falle einer Austauschbarkeit kann je nach Benutzer ein unterschiedliches Duft abgebendes Element eingesetzt sein. Durchaus besteht auch die Möglichkeit ein solches Duft abgebendes Element als Teil des Haarformoder Haarpflegegerätes zu integrieren, ohne dass ein Austausch möglich ist.

Der von dem Gerät geförderte Warmluftstrom weist über sein Querschnittsprofil hinweg eine inhomogene Temperaturverteilung auf, so dass Zonen tieferer Temperaturen und zumindest eine Zone höherer Temperatur gegeben ist. Diese Zonen bilden typischerweise Ringstrukturen. In einem solchen Fall bildet eine Zone höherer Temperatur eine Ringstruktur, an die sich nach außen und nach innen hin Zonen tieferer Temperaturen anschließen. Der Übergang zwischen den einzelnen Zonen ist kontinuierlich. Das Duft abgebende Element ist innerhalb dieses bezüglich seiner Temperaturverteilung uneinheitlichen Warmluftstroms entsprechend der gewünschten Duftabgabemenge angeordnet. Dabei macht man sich zunutze, dass die Duftabgabemenge des Duft abgebenden Elementes bei steigenden Temperaturen zunimmt, bei fallenden Temperaturen dagegen abnimmt. Somit ist bei höheren Temperaturen die Duftabgabemenge größer als bei geringeren Temperaturen. Die Geruchsintensität des austretenden Warmluftstroms kann als Maß für die abgegebene Duftmenge dienen.

Angeordnet ist das Duft abgebende Element innerhalb des bezüglich seines Querschnittsprofils temperaturinhomogenen Warmluftstroms dergestalt, das bei einem Fördern des Warmluftstroms von dem Duft abgebenden Element die gewünschte Duftmenge abgegeben wird. Befindet sich das Duft abgebende Element im Bereich der Zone maximaler Temperatur des Warmluftstroms, wird die höchste Duftabgaberate erzielt werden. Befindet sich das Duft abgebende Element dagegen in Zonen tieferer Temperaturen oder auch nur teilweise in Zonen tieferer Temperaturen ist die Duftabgaberate beim Fördern des Warmluftstroms geringer. Auf diese Weise ist die Duftabgabemenge in Abhängigkeit von der Anordnung des Duft abgebenden Elementes innerhalb des Warmluftstroms einrichtbar bzw. einstellbar. Zum Realisieren dieses Konzeptes kann das Duft abgebende Element selbst gegenüber seinem Halter eingerichtet werden, um die Duftabgabemenge zu ändern. Zu diesem Zweck kann das Duft abgebende Element, zweckmäßigerweise als Ringkörper konzipiert, exzentrisch zur Längsachse des Warmluftstroms drehbewegbar gehalten sein. Des Weiteren ist es beispielsweise möglich, das Duft abgebende Element bei einem Gerät mit einem sich zum Luftaustritt hin konisch verjüngenden Luftaustrittskanal in Längserstreckung des Luftaustrittskanals einrichtbar zu konzipieren. Die Erfindung wird auch realisiert, wenn ein Duft abgebendes Element eine fixe Anordnung innerhalb des temperaturuneinheitlichen Warmluftstroms aufweist, da auch bei einer solchen Ausgestaltung das Duft abgebende Element hinsichtlich seiner Anordnung zu dem Warmluftstrom dergestalt angeordnet sein wird, dass beim Fördern des Warmluftstroms eine vorbestimmte Duftabgaberate erzielt wird.

Nachfolgend ist die Erfindung anhand eines Ausführungsbeispiels unter Bezugnahme auf die beigefügten Figuren beschrieben. Es zeigen:
- **Fig. 1:**: eine schematisierte Drauf- bzw. Einsicht von oben auf bzw. in einen Haartrockner,
- **Fig. 2:**: eine Frontansicht des Haartrockners der Figur 1,
- **Fig. 3:**: ein Haartrockner gemäß einer weiteren Ausgestaltung in einer schematisierten Frontansicht mit einem Duft abgebenden Ringkörper in einer ersten Stellung zusammen mit einem Temperaturprofil des geförderten Warmluftstroms,
- **Fig. 4:**: der Haartrockner der Figur 3 mit seinem Duft abgebenden Element in einer weiteren Stellung,
- **Fig. 5:**: eine schematisierte Längsschnittdarstellung des Luftsaustrittskanals eines Haartrockners gemäß einer weiteren Ausgestaltung mit einem darin angeordneten Duft abgebenden Element in einer ersten Stellung und
- **Fig. 6:**: der Haartrockner der Figur 5 mit seinen Duft abgebenden Elementen einer weiteren Stellung.

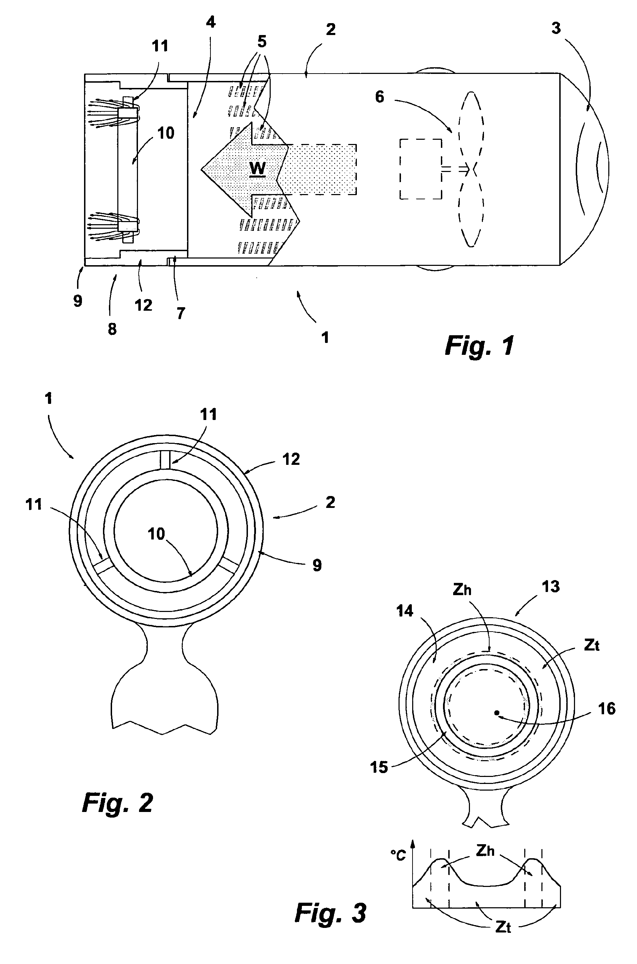

Ein elektrischer Haartrockner 1 umfasst ein Lüftergehäuse 2 mit einem Lufteinlass 3 und einem Luftaustrittskanal 4. Der Lufteinlass 3 ist nach Art eines Gitters ausgebildet. Ein im Bereich des Luftaustrittskanals 4 befindlicher Eingriffschutz ist bei diesem oder den weiteren dargestellten Ausführungsbeispielen der Übersicht halber nicht dargestellt. In dem Lüftergehäuse 2 befinden sich in einer ringförmigen Anordnung mehrere Widerstandsheizspiralen 5, die einen Strömungskanal einschließen. Die bei dem dargestellten Ausführungsbeispiel als Widerstandsheizelemente eingesetzten Widerstandsheizspiralen 5 sind mit Abstand zu der Innenseite des Lüftergehäuses 2 angeordnet. Ferner befindet sich neben anderen, in den Figuren nicht dargestellten Elementen in dem Lüftergehäuse 2 des Haartrockners 1 ein Axialventilator 6 zum Fördern eines Luftstromes. Die Förderrichtung des bei einer Bestromung der Widerstandsheizspiralen 5 geförderten Warmluftstroms W ist in Figur 1 durch einen Blockpfeil schematisiert dargestellt.

In dem Luftaustrittskanal 4 des Haartrockners 1 ist mit seinem Verbindungsabschnitt 7 ein ringförmiger Kupplungsring 8 eingesetzt. Der in Strömungsrichtung eines geförderten Luftstroms W abströmseitige Abschluss 9 des Kupplungsringes 8 ist ausgebildet wie der Abschluss des Luftaustrittskanals 4, so dass Aufsätze, wie beispielsweise Düsen oder dergleichen gleichermaßen auf den Abschluss 9 des Kupplungsringes 8 aufgesteckt werden können. Der Kupplungsring 8 trägt ein als Ringkörper ausgebildetes Kunststoffformteil 10 als Duft abgebendes Element. Das Kunststoffformteil 10 ist über drei Stege 11 mit dem den eigentlichen Kupplungsring 8 ausbildenden Ringkörper 12 verbunden. In dem Duft abgebenden Kunststoffformteil 10 sind Duftmoleküle eingelagert in einer ausreichenden Menge, dass aus dem Kunststoffformteil 10 auch bei einer Wärmebeaufschlagung über einen längeren Zeitraum hinweg Duftmoleküle abgegeben werden können. Das Kunststoffformteil 10 ist an die Stege 11 und somit an den Ringkörper 12 angeformt, wobei es sich bei dem Kunststoffformteil 10 durchaus um einen anderen Kunststoff handeln kann als bei den Stegen 11 bzw. dem Ringkörper 12.

Das Kunststoffformteil 10 ist mit einem Duft bzw. einen Duft abgebenden Stoff dotiert, der in dem Kunststoffformteil 10 enthalten und eingeschlossen ist. Der Duft ist in dem Kunststoffformteil 10 nicht gekapselt; vielmehr treten aus dem Kunststoffformteil 10, insbesondere bei einer Temperaturbeaufschlagung sensorisch aktive Duftmoleküle aus. Besonders begünstigt ist die Abgabe von Duftmolekülen, wenn ein Warmluftstrom das Kunststoffformteil 10 anströmt. Die Wärmezufuhr hat zur Folge, dass die Duftmoleküle aus dem als Duft abgebenden Element dienenden Kunststoffformteil 10 ausgeschwitzt werden. Die Abgabe der Duftmoleküle ist in Figur 1 bei gefördertem Luftstrom schematisiert im Bereich der Schnittebene dargestellt.

Der innerhalb des Luftaustrittskanals 4 des Lüftergehäuses 2 geförderte Warmluftstrom W weist über sein Querschnittsprofil hin eine inhomogene Temperaturverteilung auf. Eine Zone maximaler Temperatur befindet sich bei dem dargestellten Ausführungsbeispiels innerhalb des äußeren Drittels des geförderten Warmluftstroms W, von dem nach außen und nach innen hin die Temperatur abnimmt. Das Kunststoffformteil 10 befindet sich bei den Ausführungsbeispielen gemäß Figuren 1 und 2 in der Zone der maximalen Temperatur des geförderten Warmluftstroms, so dass bei einem Betrieb des Haartrockners 1 zum Fördern eines Warmluftstroms W eine maximale Duftabgaberate erreicht wird.

Die geringe Größe der von dem Kunststoffformteil 10 abgegebenen Duftmoleküle begünstigen ihre Aufnahme in den geförderten Luftstrom und ihre Vermischung darin, wobei auftretende Turbulenzen einen solchen Vermischungsvorgang begünstigen. Mithin stellt der geförderte Luftstrom das Transportmedium zum Transportieren der Duftmoleküle in das Haar dar.

Figur 2 zeigt schematisiert nochmals den Aufbau des Kupplungsringes 8 und die Anordnung des Duft abgebenden Kunststoffformteils 10 innerhalb des Ringkörpers 12.

Figur 3 zeigt einen weiteren Haartrockner 13 in einer Frontansicht und einem in seinem Luftsaustrittskanal 14 angeordneten, als Ringkörper ausgebildeten Duft abgebenden Element 15. Das Duft abgebende Element 15 ist grundsätzlich ebenso konzipiert wie das Duft abgebende Element 10 der Figuren 1 und 2. Das Duft abgebende Element 15 ist innerhalb des Luftaustrittskanals 14 exzentrisch um eine parallel zur Längsachse des Luftaustrittskanals 14 versetzt angeordnete Drehachse 16 verstellbar. In der in Figur 3 gezeigten Stellung des Duft abgebenden Elements 15 befindet sich dieses im Bereich der Zone Zₕ maximaler Temperatur des geförderten Warmluftstroms W. Von dieser Zone Zₕ nimmt die Temperatur nach außen zum Lüftergehäuse und nach innen zum Zentrum des Warmluftstroms W hin ab. Die Temperaturverteilungskurve des durch den Haartrockner 13 generierten Warmluftstroms W ist in Figur 3 wiedergegeben. Die Temperaturverteilungskurve macht deutlich, dass sich die Temperaturen innerhalb des Warmluftstroms W kontinuierlich und nicht sprunghaft ändern.

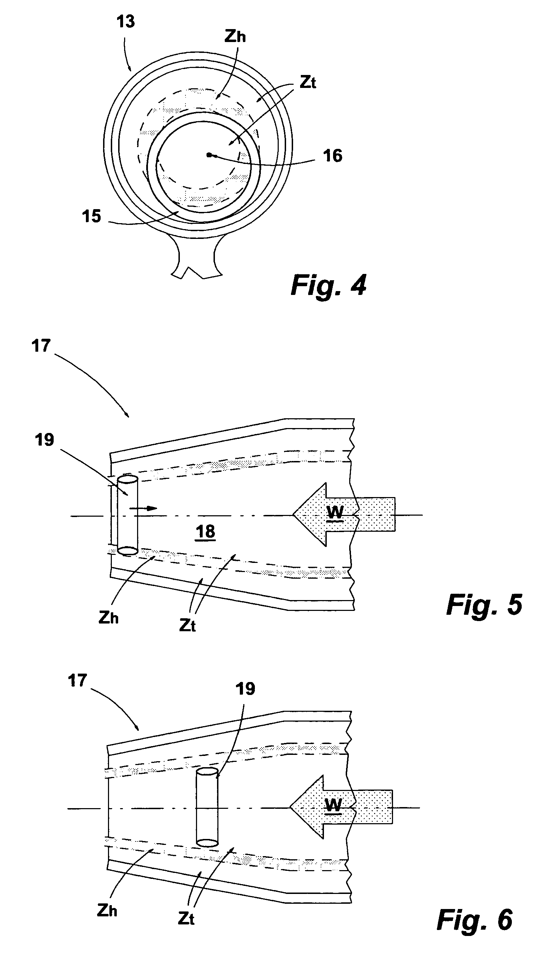

Das verstellbare Duft abgebende Element 15 ist in Figur 4 in einer seiner anderen Stellungen bezüglich des geförderten Warmluftstroms W und insbesondere bezüglich seiner Zone Zₕ maximaler Temperaturen dargestellt. In dieser Stellung befinden sich lediglich zwei Ringsegmente des Duft abgebenden Elementes 15 in der Zone Zₕ höherer Temperaturen, während die anderen Ringsegmente in Zonen Zₜ tieferer Temperaturen liegen. Während bei der Stellung des Duft abgebenden Elementes 15 in Figur 3, in der sich dieses insgesamt in der Zone Zₕ höherer Temperaturen des Warmluftstroms W befindet und in dieser Stellung eine maximale Duftabgaberate erfolgt, ist die Duftabgaberate bzw. die abgegebene Duftmenge bei der in Figur 4 gezeigten Stellung des Duft abgebenden Elementes 15 geringer, da in den kühleren Ringsegmenten des Duft abgebenden Elementes 15 die Duftabgabemenge geringer ist als im Bereich derjenigen Ringsegmente, die von wärmeren Teilen des geförderten Warmluftstroms W angeströmt werden. Folglich ist die Duftabgabemenge des Duft abgebenden Elementes 15 in seiner in Figur 4 gezeigten Stellung geringer.

Figuren 5 und 6 zeigen einen weiteren Haartrockner 17, dessen Luftaustrittskanal 18 sich in Förderrichtung des Warmluftstroms W hin verjüngt. Entsprechend verhält sich auch der geförderte Warmluftstrom W bzw. seine Zone Zₕ höherer Temperaturen. Das Duft abgebende Element 19 des Haartrockners 17 ist auch bei diesem Ausführungsbeispiel als Ringkörper ausgebildet. Das Duft abgebende Element 15 ist in Richtung der Längsachse des Luftauslasskanals in nicht näher dargestellten Art und Weise verschiebbar, so dass sich dieses in seiner einen, in Figur 5 gezeigten Endstellung innerhalb der Zone Zₕ höherer Temperaturen des geförderten Warmluftstroms W befindet. Figur 6 zeigt das Duft abgebende Element 19 in seiner anderen Endstellung, in der sich diese vollständig außerhalb der Zone Zₜ tieferer Temperaturen befindet und in welcher Stellung folglich die Duftabgaberate des Duft abgebenden Elementes 19 geringer ist als in der in Figur 5 gezeigten Stellung.

Die Beschreibung der Erfindung, die beispielhaft anhand der in den Figuren gezeigten Ausführungsbeispiele vorgenommen worden ist, macht deutlich, dass ohne besondere Maßnahmen eine Einstellbarkeit der Duftabgaberate eines Duft abgebenden Elementes im Bereich des Luftaustrittskanals eines Haarform- oder Haarpflegegerätes möglich ist, indem ein bezüglich seiner Temperaturverteilung entlang seines Querschnittprofils inhomogener Warmluftstrom generiert wird und indem das Duft abgebende Element in Abhängigkeit von seiner Anordnung innerhalb des geförderten Warmluftstroms mehr oder weniger Duft abgibt, wobei das Duft abgebende Element konzipiert ist, um bei höheren Temperaturen eine größere Duftmenge abzugeben als bei geringeren Temperaturen.

### Bezugszeichenliste

- 1: Haartrockner
- 2: Lüftergehäuse
- 3: Lufteinlass
- 4: Luftaustrittsöffnung
- 5: Widerstandsheizspirale
- 6: Axialventilator
- 7: Verbindungsabschnitt
- 8: Kupplungsring
- 9: Abschluss
- 10: Kunststoffformteil, Duft abgebendes Element
- 11: Steg
- 12: Ringkörper
- 13: Haartrockner
- 14: Luftaustrittskanal
- 15: Duft abgebendes Element
- 16: Drehachse
- 17: Haartrockner
- 18: Luftaustrittskanal
- 19: Duft abgebendes Element

- W: Warmluftstrom
- Zₕ: Zone höherer Temperaturen
- Zt: Zone tieferer Temperaturen

## Patentansprüche

1. Elektrisches Haarform- oder Haarpflegegerät mit einem Warmluftgebläse (6) zum Fördern eines für eine Haarbehandlung - Trocknen und/oder Formen - notwendigen Warmluftstroms (W), wobei das Gerät (1, 13, 17) ein oder mehrere, von dem geförderten Luftstrom (W) angeströmte, Duft abgebende Elemente (10, 15, 19) aufweist und die von einem solchen Element (10, 15, 19) bei einem Betrieb des Gerätes (1, 13, 17) abgegebenen, sensorisch aktiven Moleküle von dem geförderten Luftstrom (W) aufgenommen und zum Haar transportiert werden, **dadurch gekennzeichnet, dass** das Temperaturprofil innerhalb des geförderten Warmluftstroms (W) über sein Querschnittsprofil hinweg im Bereich des Duft abgebenden Elements (10, 15, 19) ein oder mehrere Zonen (Zₜ) tieferer Temperaturen und zumindest eine Zone (Zₕ) höherer Temperatur aufweist, dass die Duftabgabemenge des Duft abgebenden Elements (10; 15, 17) bei steigenden Temperaturen zunimmt und das Duft abgebende Element (10, 15, 17) ausgebildet ist, damit in Abhängigkeit von der Anordnung des Duft abgebenden Elements (10, 15, 17) innerhalb des geförderten Warmluftstroms (W) unter Ausnutzung der unterschiedlichen Temperaturzonen (Zₜ, Zₕ) des Warmluftstroms (W) die Duftabgabemenge unterschiedlich ist.

2. Haarform- oder Haarpflegegerät nach Anspruch 1, **dadurch gekennzeichnet, dass** das Duft abgebende Element (10, 15, 17) ein Ringkörper ist.

3. Haarform- oder Haarpflegegerät nach Anspruch 1 oder 2, **dadurch gekennzeichnet, dass** das zumindest eine Duft abgebende Element ein Formteil, insbesondere ein Kunststoffformteil (10, 15, 19) oder Teil eines solchen Formteils ist.

4. Haarform- oder Haarpflegegerät nach Anspruch 3, **dadurch gekennzeichnet, dass** das Formteil ein auf die Luftaustrittsöffnung des Warmluftgebläses aufsetzbarer Ringkörper oder ein Teil desselben ist.

5. Haarform- oder Haarpflegegerät nach Anspruch 3, **dadurch gekennzeichnet, dass** das Formteil (10) ein auf die Luftaustrittsöffnung des Warmluftgebläses aufsetzbarer, als ein Kupplungsstück konzipierter Ringkörper oder ein Teil desselben ist.

6. Haarform- oder Haarpflegegerät nach Anspruch 5, **dadurch gekennzeichnet, dass** das Kupplungsstück (8) einen äußeren, das Kupplungsstück bildenden Ringkörper (12) mit nach innen abragenden Stegen (11) aufweist, an denen das Duft abgebende Element (10), etwa als ringförmiges Formteil ausgebildet, gehalten ist.

7. Haarform- oder Haarpflegegerät nach Anspruch 1, **dadurch gekennzeichnet, dass** das Warmluftgebläse des Gerätes (1, 13, 17) einen Warmluftstrom (W) fördert, der über seinen Querschnitt hin ein Temperaturprofil aufweist, bei dem sich das Temperaturmaximum innerhalb einer von der fiktiven Mantelfläche des Warmluftstroms (W) beabstandeten Zone (Zₕ) befindet und die Temperatur von dieser Zone (Zₕ) nach außen und nach innen hin abnimmt.

8. Haarform- oder Haarpflegegerät nach Anspruch 7, **dadurch gekennzeichnet, dass** sich die Zone des Temperaturmaximums (Zₕ) innerhalb der äußeren Hälfte des Radius des geförderten Warmluftstroms (W) befindet.

9. Haarform- oder Haarpflegegerät nach einem der Ansprüche 1 bis 8, **dadurch gekennzeichnet, dass** das Duft abgebende Element (15) um eine parallel zur Längsachse des Warmluftstroms (W) und versetzt zu dieser angeordnete Drehachse (16) verstellbar ist.

10. Haarform- oder Haarpflegegerät nach einem der Ansprüche 1 bis 8, **dadurch gekennzeichnet, dass** der Luftaustrittskanal (18) des Gerätes (17) sich zum Luftaustritt hin verjüngt und dass Duft abgebende Element (19) innerhalb des verjüngten Luftaustrittskanals (18) entlang der Längsachse des Luftaustrittskanals (18) einrichtbar ist.

## Claims

1. Electric appliance for shaping hair or for hair care, the said appliance having a hot air blower (6) for conveying a hot air flow (W) that is necessary for a hair treatment - drying and/or shaping -, wherein the : appliance (1, 13, 17) includes one or more elements (10, 15, 19) that emit a pleasant scent and are traversed by the conveyed air flow (W) and the molecules, which are emitted by such an element (10, 15, 19) when the appliance (1, 13, 17) is in operation and are active in a sensory manner, are picked up by the conveyed air flow (W) and transported to the hair, **characterised in that** the temperature profile internally of the conveyed hot air flow (W) includes one or more zones (Zₜ) of lower temperatures and at least one zone (Zₕ) of a higher temperature beyond its cross-sectional profile in the region of the element (10, 15, 19) that emits a pleasant scent, **in that** the amount of scent emitted by the element (10; 15, 17) that emits a pleasant scent increases as the temperatures increase and the element (10, 15, 17) that emits the pleasant scent is designed so that, in dependence on the disposition of the element (10, 15, 17) that emits the pleasant scent internally of the conveyed hot air flow (W), by utilising the different temperature zones (Zₜ, Zₕ) of the hot air flow (W), the amount of pleasant scent emitted is variable.

2. Appliance for shaping hair or for hair care according to claim 1, **characterised in that** the element (10, 15, 17) that emits the pleasant scent is an annular body.

3. Appliance for shaping hair or for hair care according to claim 1 or 2, **characterised in that** the element that emits at least one pleasant scent is a moulded part, more especially a plastics material moulded part (10, 15, 19) or part of such a moulded part.

4. Appliance for shaping hair or for hair care according to claim 3, **characterised in that** the moulded part is an annular body that can be placed onto the air outlet opening of the hot air blower or a part of such an annular body.

5. Appliance for shaping hair or for hair care according to claim 3, **characterised in that** the moulded part (10) is an annular body that can be placed onto the air outlet opening of the hot air blower and is designed in the form of a coupling piece or is a part of such an annular body.

6. Appliance for shaping hair or for hair care according to claim 5, **characterised in that** the coupling piece (8) includes an outer annular body (12) that forms the coupling piece with webs (11) that extend inwards, the element (10) that emits the pleasant scent, for example in the form of a ring-shaped moulded part, being retained on the said webs.

7. Appliance for shaping hair or for hair care according to claim 1, **characterised in that** the hot air blower of the appliance (1, 13, 17) conveys a hot air flow (W), which, beyond its cross-section, has a temperature profile where the maximum temperature is inside a zone (Zₕ) at a spacing from the imaginary outside surface of the hot air flow (W) and the temperature of the said zone (Zₕ) reduces towards the outside and towards the inside.

8. Appliance for shaping hair or for hair care according to claim 7, **characterised in that** the zone of the maximum temperature (Zₕ) is situated inside the outside half of the radius of the conveyed hot air flow (W).

9. Appliance for shaping hair or for hair care according to one of claims 1 to 8, **characterised in that** the element (15) that emits the pleasant scent is adjustable about an axis of rotation (16) that is disposed parallel to the longitudinal axis of the hot air flow (W) and is offset relative to the said longitudinal axis.

10. Appliance for shaping hair or for hair care according to one of claims 1 to 8, **characterised in that** the air outlet duct (18) of the appliance (17) is tapered towards the air outlet and **in that** the element (19) that emits the pleasant scent can be set up inside the tapered air outlet duct (18) along the longitudinal axis of the air outlet duct (18).

## Revendications

1. Appareil électrique de mise en forme ou de soin des cheveux comprenant une soufflerie d'air chaud (6) destinée à refouler un flux d'air chaud (W) indispensable pour le traitement - séchage et/ou mise en forme - des cheveux, l'appareil (1, 13, 17) présentant un ou plusieurs éléments (10, 15, 19) diffusant une odeur, traversés par le flux d'air (W) refoulé et dont les molécules sensoriellement actives diffusées par un tel élément (10, 15, 19) lors du fonctionnement de l'appareil (1, 13, 17) sont emportées par le flux d'air (W) refoulé et transportées jusqu'aux cheveux **caractérisé en ce que** le profil des températures du flux d'air chaud (W) refoulé présente sur tout son profil en travers, dans la zone des éléments (10, 15, 19) diffusant une odeur, une ou plusieurs zones (Zₜ) de températures plus basses et au moins une zone (Zₕ) de températures plus élevées, pour lequel la quantité d'odeur diffusée par l'élément (10, 15, 17) diffusant une odeur augmente au fur et à mesure de l'accroissement des températures et pour lequel l'élément (10, 15, 17) diffusant une odeur est conformé de façon à ce que la quantité d'odeur diffusée est différente en fonction de la disposition de l'élément (10, 15, 17) diffusant une odeur dans le flux d'air chaud (W) refoulé en exploitant les différentes zones de températures (Zₜ, Zₕ) du flux d'air chaud (W).

2. Appareil de mise en forme ou de soin des cheveux selon la revendication 1 **caractérisé en ce que** l'élément (10, 15, 17) diffusant une odeur est un corps annulaire.

3. Appareil de mise en forme ou de soin des cheveux selon la revendication 1 ou 2 **caractérisé en ce que** l'au moins un élément diffusant une odeur est une pièce moulée, notamment une pièce moulée en matière plastique (10, 15, 19) ou une partie d'une telle pièce moulée.

4. Appareil de mise en forme ou de soin des cheveux selon la revendication 3 **caractérisé en ce que** la pièce moulée est un corps annulaire enfichable sur une ouverture de sortie d'air de la soufflerie d'air chaud ou une partie de celui-ci.

5. Appareil de mise en forme ou de soin des cheveux selon la revendication 3 **caractérisé en ce que** la pièce moulée (10) est un corps annulaire conçu comme une pièce de couplage enfichable sur la sortie d'air chaud de la soufflerie d'air chaud ou une partie de celui-ci.

6. Appareil de mise en forme ou de soin des cheveux selon la revendication 5 **caractérisé en ce que** la pièce de couplage (8) présente vers l'extérieur un corps annulaire (12) formant la pièce de couplage et vers l'intérieur des entretoises (11) en saillie à l'aide desquelles l'élément (10) diffusant une odeur, conformé par exemple en pièce moulée de forme annulaire, est maintenu.

7. Appareil de mise en forme ou de soin des cheveux selon la revendication 1 **caractérisé en ce que** la soufflerie d'air chaud de l'appareil (1, 13, 17) refoule un flux d'air chaud (W), qui présente sur sa section un profil de températures où la température maximale se situe dans une zone (Zₕ) à distance de la surface périphérique fictive du flux d'air chaud (W) et dont la température dans cette zone (Zₕ) diminue vers l'extérieur et vers l'intérieur.

8. Appareil de mise en forme ou de soin des cheveux selon la revendication 7 **caractérisé en ce que** la zone des températures maximales (Zₕ) se trouve dans la moitié extérieure du rayon du flux d'air chaud (W) refoulé.

9. Appareil de mise en forme ou de soin des cheveux selon l'une des revendications 1 à 8 **caractérisé en ce que** l'élément (15) diffusant une odeur est réglable sur un axe de rotation (16) parallèle à l'axe longitudinal du flux d'air chaud (W) et disposé avec un décalage par rapport ce dernier.

10. Appareil de mise en forme ou de soin des cheveux selon l'une des revendications 1 à 8 **caractérisé en ce que** le canal de sortie d'air (18) de l'appareil (17) se rétrécit vers la sortie d'air et **en ce que** l'élément (19) diffusant une odeur est ajustable au niveau du canal de sortie d'air (18) rétréci, le long de l'axe longitudinal du canal de sortie d'air (18).
